# FASCICULE DE BREVET EUROPEEN

(11) **EP 0 956 022 B1**
(45) Date de publication et mention de la délivrance du brevet: **23.04.2003**
(21) Numéro de dépôt: 97944940.2
(22) Date de dépôt: 08.10.1997
(51) Int. Cl.: A61K 31/57

(54) **COMPOSITION HORMONALE CONSTITUEE D'UN COMPOSE ESTROGENE ET D'UN COMPOSE PROGESTATIF**
HORMONZUSAMMENSETZUNG WELCHE EIN ÖSTROGENES MITTEL UND EIN PROGESTATIVES MITTEL ENTHÄLT
HORMONAL COMPOSITION CONSISTING OF AN OESTROGEN COMPOUND AND OF A PROGESTATIONAL COMPOUND

(30) Priorité: 08.10.1996 FR 9612239
(43) Date de publication de la demande: 17.11.1999
(73) Titulaire: LABORATOIRE THERAMEX, 98000 Monaco (MC)
(72) Inventeur: LANQUETIN, Michel, F-06340 La Trinité (FR); PARIS, Jacques, Le Clos de Cimiez, F-06100 Nice (FR); THOMAS, Jean-Louis, F-94220 Charenton le Pont (FR)
(74) Mandataire: Burtin, Jean-François
(86) Numéro de dépôt international: FR9701792
(87) Numéro de publication internationale: WO98015279

(56) Documents cités:
- CONARD J ET AL: "Cardiovascular risk factors and combined estrogen-progestin replacement therapy: A placebo-controlled study with nomegestrol acetate and estradiol" FERTILITY AND STERILITY, 64 (5). 1995. 957-962., XP000675866
- SITRUK-WARE R: "[Pharmacology of oral contraceptives]" REV PRAT, DEC 1 1995, 45 (19) P2401-6, FRANCE, XP000675852

## Description

La présente invention se rapporte au domaine de la chimie thérapeutique et plus particulièrement au domaine de la technique pharmaceutique hormonale.

Elle a plus précisément pour objet l'utilisation de compositions pharmaceutiques formées d'une association estroprogestative selon les revendication 1-10 en vue de la correction des carences estrogéniques dans les ménopauses naturelles ou artificielles ou afin de bloquer l'ovulation chez la femme en période d'activité ovarienne.

Une association estroprogestative est utilisée, caracterisée en ce qu'elle est constituée par des unités de dosage renfermant la combinaison d'un progestatif et d'un estrogène, les deux composants étant présents simultanément dans chaque prise médicamenteuse.

Cette association est destinée à être administrée par voie orale.

Comme on le sait, l'espérance de vie de la femme est passée en moins d'un siècle de 50 à 80 ans, tandis que l'âge moyen de survenue de la ménopause est resté inchangé. Ainsi, les femmes passent le tiers de leur vie en état de carence estrogénique ce qui est à l'origine de l'augmentation du risque d'ostéoporose et de maladies cardio-vasculaires.
Le traitement substitutif séquentiel de la ménopause guérit la symptomatologie climatérique et prévient l'ostéoporose et la survenue des maladies cardiovasculaires. Il créé des cycles artificiels qui sont suivis par une hémorragie de privation. Ce schéma thérapeutique convient tout particulièrement aux femmes dont la ménopause est récente mais il n'est pas toujours bien accepté à long terme, ce qui explique en partie la médiocre observance du traitement (DRAPIER FAURE E.; Gynécologie, 1992, 43: 271-280 ).

Pour pallier cet inconvénient, on a mis au point des associations combinées où les deux composants sont pris simultanément, le progestatif ayant pour effet de s'opposer en permanence à l'action proliférative de l'estrogène sur l'endomètre, en créant une atrophie de l'endomètre et par voie de conséquence, l'absence d'hémorragie de privation (HARGROVE J.T., MAXSON W.S., WENTZ A.C., BURNETT L.S, Obstet Gynecol, 1989, 73 : 606-612 ).

Ce schéma « sans règles » convient plus particulièrement aux femmes dont la ménopause est déjà ancienne. Il peut être prescrit en relais des associations séquentielles afin d'améliorer l'observance au long cours du traitement hormonal substitutif de la ménopause.

La dose de progestatif à utiliser dans un traitement substitutif combiné est en général déduite de celle qui est habituellement prescrite dans les schémas séquentiels. Dans ces derniers la dose choisie est celle qui donne à long terme moins de 1 % d'hyperplasie endométriale lorsque le progestatif est administré en discontinu, plus de 10 jours par cycle, chez les femmes ménopausées sous estrogénothérapie substitutive (WHITEHEAD et coll., J. Reprod. Med, 1982, 27 : 539-548, PATERSON et coll., Br Med J, 1980, 22 March : 822-824).

Dans le traitement combiné, ces mêmes progestatifs ont été utilisés à la moitié de la dose jugée efficace lors d'un traitement séquentiel : c'est l'exemple de la progestérone micronisée, de la didrogestérone (FOX H., BAAK J., VAN DE WEIJER P., AL-AZZAWI E., PATERSON M., JOHNSON A., MICHELL G., BARLOW D., FRANCIS R., 7 th International Congress on the Menopause, Stockholm, 20-24 Juin 1993, abstr 119) et de l'acétate de médroxyprogestérone (BOCANERA R., BEN J., COFONE M., GUINLE I., MAILAND D., SOSA M., POUDES G., ROBERTI A., BISO T., EZPELETA D., PUCHE R., TOZZINI R., 7 th International Congress of the Menopause, Stockholm, 20-24 Juin 1993, abstr 40) qui ont été utilisées respectivement à la posologie de 100, 10 et 5 mg/jour, avec des résultats encourageants sur le plan clinique et endométrial.
Parmi les progestatifs, l'acétate de nomégestrol est apparu comme un de ceux les plus efficaces. L'acétate de nomégestrol est un progestatif non androgénique dérivé de la 19-nor progestérone, son utilisation en administration séquentielle lors de la ménopause à la dose de 5 mg/jour, 12 jours par cycle, en association avec différents types d'estrogènes, permet de prévenir l'hyperplasie endométriale comme l'a montré une étude multicentrique sur 150 femmes durant 1 an (THOMAS J.L, BERNARD A.M., DENIS C, 7 th International Congress on the Menopause, Stockolm, 20-24 Juin 1993, abstr 372 )

L'absence d'hyperplasie a été confirmée dans une étude où l'acétate de nomégestrol a été administré à la même dose, 14 jours par cycle, chez des femmes traitées par l'estradiol percutané (BERNARD A.M. et al. Comparative évaluation of two percutaneous estradiol gels in combination with nomegestrol acetate in hormone replacement therapy. XIV World Congress of Gynecology and Obstetries, FIGO, Montréal, 24-30 September 1994).

Le traitement combiné est le plus souvent utilisé de façon continue, c'est-à-dire sans interruption. Certains sont cependant partisans de l'utiliser de façon intermittente, par exemple 25 jours par mois (BIRKAUSER M et al ; Substitution hormonale : une indication bien posée et des schémas de traitement individuels sont déterminants pour le succès du traitement, Méd et Hyg, 1995, 53 : 1770-1773). L'interruption thérapeutique a pour but de lever l'inhibition exercée par le progestatif sur la synthèse des récepteurs de l'estradiol et de la progestérone et d'éviter ainsi la baisse de réceptivité des tissus hormono-dépendants.

Le progestatif utilisé est l'acétate de nomégestrol qui est actif par voie orale.
L'estrogène utilisé est l'estradiol libre ou estérifié, ou des estrogènes conjugués équins se présentant selon une formulation active par voie orale, et notamment le valérate d'estradiol.
L'acétate de nomégestrol et l'estradiol libre ou estérifié, ou les estrogènes conjugués équins sont administrés sous une des formes permettant l'administration par voie orale : gélules, capsules, pilules, sachets de poudre, comprimés, comprimés enrobés, dragées etc...

La présente invention a également pour objet son utilisation dans la correction des carences estrogéniques, dans la prévention de l'ostéoporose et des maladies cardio-vasculaires chez la femme ménopausée selon les revendications 1-10.

Les compositions, à base d'acétate de nomégestrol et d'estradiol libre ou estérifié, ou d'estrogènes conjugués équins sont administrés de façon continue ou intermittente, de 21 à 25 jours par mois.

Selon un mode d'exécution particulier, les compositions contiennent une quantité d'acétate de nomégestrol s'échelonnant de 1,5 à 3,75 mg et une quantité d'estradiol libre ou estérifié, ou d'estrogènes conjugués équins s'échelonnant de 0,5 à 3 mg. De préférence, les formulations optimales contiennent 2,5 mg d'acétate de nomégestrol associé à : soit 1,5 mg d'estradiol libre ou 2 mg d'ester d'estradiol ou 0,625 mg d'estrogènes conjugués équins, par prise journalière.

Ce mode d'administration combiné peut avoir plusieurs indications thérapeutiques. Chez les femmes ménopausées, la combinaison estroprogestative est destinée à compenser les troubles fonctionnels entraînés par l'hypoestrogénie de la ménopause, tout en maintenant une atrophie de l'endomètre et en évitant chez une majorité d'entre elles l'apparition d'hémorragie de privation.

Chez les femmes en période d'activité ovarienne, jeunes ou dans les années précédant la ménopause, l'administration cyclique de la combinaison hormonale est capable d'inhiber l'ovulation et d'exercer un effet contraceptif dans la mesure où il a été prouvé que l'acétate de nomégestrol était capable d'inhiber le pic ovulatoire de LH et de FSH, à partir de 1,25 mg/jour (BAZIN B. et al, Effect of nomegestrol acetate, a new 19-norprogesterone derivative on pituitary ovarian function in women. Br. J. Obstet. Gynaecol, 1987, 94 : 1199-1204). Lorsque la combinaison hormonale est donnée dans un but contraceptif, l'acétate de nomégestrol a pour but de bloquer l'ovulation et la composante estrogénique de compenser l'hypoestrogénie et d'assurer un meilleur contrôle du cycle.

Le procédé d'obtention consiste à mélanger les principes actifs : acétate de nomégestrol et estradiol libre ou estérifié, ou des estrogènes conjugués équins avec un ou des excipients inertes, non toxiques, pharmaceutiquement acceptables.

Parmi les excipients on pourra citer les agents liants et solubilisants, les agents de compression, les agents de désintégration et les agents de glissement.
On peut soumettre ce mélange à une compression directe ou en plusieurs étapes pour former des comprimés que l'on peut protéger en surface, si désiré par pelliculage, laquage ou enrobage. La production de comprimés par compression directe permet de réduire au maximum la proportion d'agents de dilution, d'agents liants, d'agents de désintégration et d'agents de glissement.
La production de gélules pourra se faire en mélangeant les principes actifs à un diluant inerte et un agent de glissement.
Les comprimés renferment, en particulier, des agents de dilution de la masse comme le lactose, le sorbitol pour compression directe, commercialisé sous la dénomination NEOSORB 60, le Palatinite qui est la marque déposée pour désigner un mélange équimolaire d'isomère de -D-glucopyranosido 1,6-mannitol et de -D-glucopyranosido 1,6-glucitol cristallisé avec deux molécules d'eau, la mannitol, le sorbitol ou le mélange lactose/PVP vendu sous la dénomination Ludipress.
Les agents liants de compression sont en général des celluloses microcristallines comme celles vendues sous la dénomination AVICEL PH 101 ou AVICEL PH 102.
La polyvinylpyrrolidone joue également un rôle important et facilite l'agglomération des poudres et la compressibilité de la masse. On utilise à cette fin des polyvinylpyrrolidones de poids moléculaire compris entre 10000 et 30000 comme la Povidone, le Kollidon de grade compris entre 12 et 30.
Le mélange contient également des agents de glissement ou anti-électrostatiques qui évitent que la poudre ne s'agglomère dans les trémies d'alimentation. On peut citer, à cet égard, les silices colloïdales vendues sous la dénomination AEROSIL 100 ou AEROSIL 200.

Le mélange contient aussi des agents de désintégration qui permettent une désintégration ou un délitement conforme aux normes pharmaceutiques. On pourra citer comme agents de désintégration utiles, les polymères de vinylpyrrolidones réticulées telles que celles vendues sous les dénominations Polyplasdone ou Polyclar AT, les carboxyméthylamidons comme ceux vendus sous les dénominations Amigel ou Explotab, les carboxyméthylcelluloses réticulées ou croscarmelloses comme le composé vendu sous la dénomination AC-DI-SOL.
En outre, la préparation contient des agents de lubrification qui facilitent la compression et l'éjection du comprimé sur les machines à comprimer. On pourra citer comme agents de lubrification, le palmitostéarate de glycérol vendu sous la dénomination Précirol, le stéarate de magnésium, l'acide stéarique ou le Talc.
Après compression, les comprimés peuvent être enrobés pour assurer leur conservation ou faciliter leur déglutition.
Les agents d'enrobage sont soit cellulosiques comme le phtalate de cellulose (Sepifilm, Pharmacoat), soit polyvinyliques du type Sépifilm ECL, soit saccharosiques comme le sucre pour dragéification du type Sépisperse DR, AS, AP, OU K (colorés).
Les comprimés enrobés ou non, peuvent, en outre, être colorés en surface ou dans la masse, par des colorants végétaux ou synthétiques (par ex. laque au jaune de quinoléine ou E 104).
Les proportions des différents constituants varient selon la nature du comprimé à réaliser.
La teneur en principes actifs peut varier de 1,5 à 3,75 mg pour l'acétate de nomégestrol et de 0,5 à 3 mg pour l'estradiol libre ou estérifié ou pour les estrogènes conjugués équins. Les agents de dilution varient de 20 à 75 % de la masse totale, les agents de glissement de 0,1 à 2 % de la masse totale, les agents liants de compression varient de 2 à 20 %, la polyvinylpyrrolidone de 0,5 à 15 %, les agents de désintégration varient de 2 à 5,5 % pour la polyvinylpyrrolidone réticulée ou pour le carboxyméthylamidon, de 2,0 à 3,0 % pour la croscarméllose.
Les quantités d'agents de lubrification varient en fonction de la nature de l'agent de 0,1 à 3,0 %.

Les compositions selon l'invention sont destinées à être administrées une fois par jour. Cependant, en fonction des besoins thérapeutiques, l'administration peut être fragmentée (deux fois par jour) ou bien au contraire, renouvelée (deux comprimés par jour).
Les exemples suivants illustrent l'invention. Ils ne la limitent en aucune façon.

### EXEMPLE I

### Comprimés à 4 mg de principes actifs

| | | |
|---|---|---|
| Principes actifs : | - estradiol | 1,5 mg |
| | - acétate de nomégestrol | 2,5 mg |
| Cellulose microcristalline (commercialisée sous la dénomination AVICEL PH 102) | | 22,4 mg |
| Lactose | | 60 mg |
| Polyvinylpyrrolidone | | 8,4 mg |
| Silice colloïdale | | 1,2 mg |
| Palmitostéarate de glycérol | | 3,6 mg |
| Colorant E.104 | | 0,4 mg |
| pour un comprimé terminé au poids moyen de 100 mg. | | |

### EXEMPLE II

### Etude de la tolérance clinique lors de deux schémas combinés continus d'hormonothérapie substitutive de la ménopause

L'étude pilote s'effectue durant 24 semaines sur deux groupes parallèles soumis aux traitements A et C :

### Traitement A

- Acétate de nomégestrol 2,5 mg/j tous les jours + 17β-estradiol percutané 1,5 mg/j tous les jours.
- L'acétate de nomégestrol est administré sous forme de comprimés et le 17β-estradiol percutané sous forme de gel.

### Traitement C

- Acétate de nomégestrol 2,5 mg/j tous les jours + valérate d'estradiol 2 mg/j tous les jours.
- Le valérate d'estradiol est administré sous forme de comprimés.

L'étude pilote est destinée à évaluer la tolérance clinique endométriale lors de l'utilisation des deux schémas d'hormonothérapie substitutive de la ménopause dit « sans règles », associant d'une façon combinée continue le traitement A ou C. La tolérance clinique endométriale s'évalue à partir de la présence ou non de saignements vaginaux, de leur intensité, de leur fréquence, à partir des données de l'examen échographique endovaginal etc...

Un autre but de cette étude est aussi d'apprécier la tolérance clinique générale (poids, tension artérielle, manifestations mammaires), la tolérance biologique (Numérotation Formule Sanguine, glycémie, cholestérol...), ainsi que l'observance du traitement.

La sélection des sujets s'effectue en fonction de critères « d'inclusion ». Ces critères sont en rapport :
- **avec la ménopause :**
   sont incluses les femmes de plus de 50 ans ayant eu une ménopause naturelle s'exprimant cliniquement par une aménorrhée supérieure à 12 mois et inférieure à 10 ans, les femmes ayant eu une ménopause naturelle confirmée biologiquement par le dosage de FSH (Follicle - stimulating hormone) et d'estradiol (soit FSH plasmatique ≥ à 20 UI/I, E₂ plasmatique ≤ à 0,11 nmol/l).
- **avec les femmes :**
   sont incluses les femmes non hystérectomisées, dont l'indice de Quetelet [poids en kg / (taille en m)²] est ≤ à 27, ayant eu des cycles réguliers avant la ménopause, n'ayant jamais reçu un traitement hormonal substitutif de la ménopause ou ayant eu un traitement hormonal substitutif bien toléré cliniquement (absence de saignements anormaux), interrompu depuis plus de 6 semaines, présentant une épaisseur endométriale mesurée par échographie endovaginale ≤ à 5 mm, acceptant l'idée d'une hormonothérapie substitutive de la ménopause, désireuses d'une hormonothérapie sans règles, justiciables d'une hormonothérapie estroprogestative pendant au moins 6 mois, coopérantes : acceptant de se conformer aux impératifs de l'étude, dont le profil psychique ou intellectuel laisse supposer une bonne observance du traitement, ayant une mammographie datant de moins d'un an à la date de l'inclusion.

Au début du traitement les patientes subissent une consultation d'inclusion (C₁) ayant pour but de vérifier le respect des critères d'inclusion, la normalité de l'échographie endovaginale, de recueillir le consentement de participation de la patiente par écrit.
La consultation intermédiaire (C₂) a lieu entre la 9ème à la 11ème semaine du traitement, ayant pour but de vérifier la bonne tolérance clinique endométriale et mammaire du traitement.
Enfin, une consultation finale (C₃) a lieu lors de la 24ème semaine du traitement.

Les patientes désirant poursuivre l'essai peuvent recevoir durant 24 semaines supplémentaires le traitement estroprogestatif reçu durant l'essai, selon le même schéma thérapeutique. L'extension d'essai permet ainsi un suivi total de l'essai de 48 semaines.

### ANALYSE DE L'ETUDE

### RESULTATS I

Les tableaux I et II ci-joints, mettent en évidence une différence en terme de résultats d'aménorrhée (c'est-à-dire, aucun saignement de 0 à 24 semaines) et de tolérance mammaire et/ou endométriale en fonction de l'estrogène.

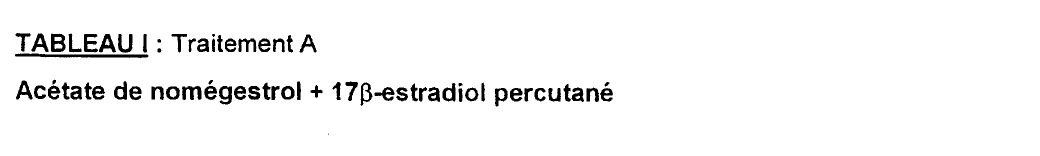

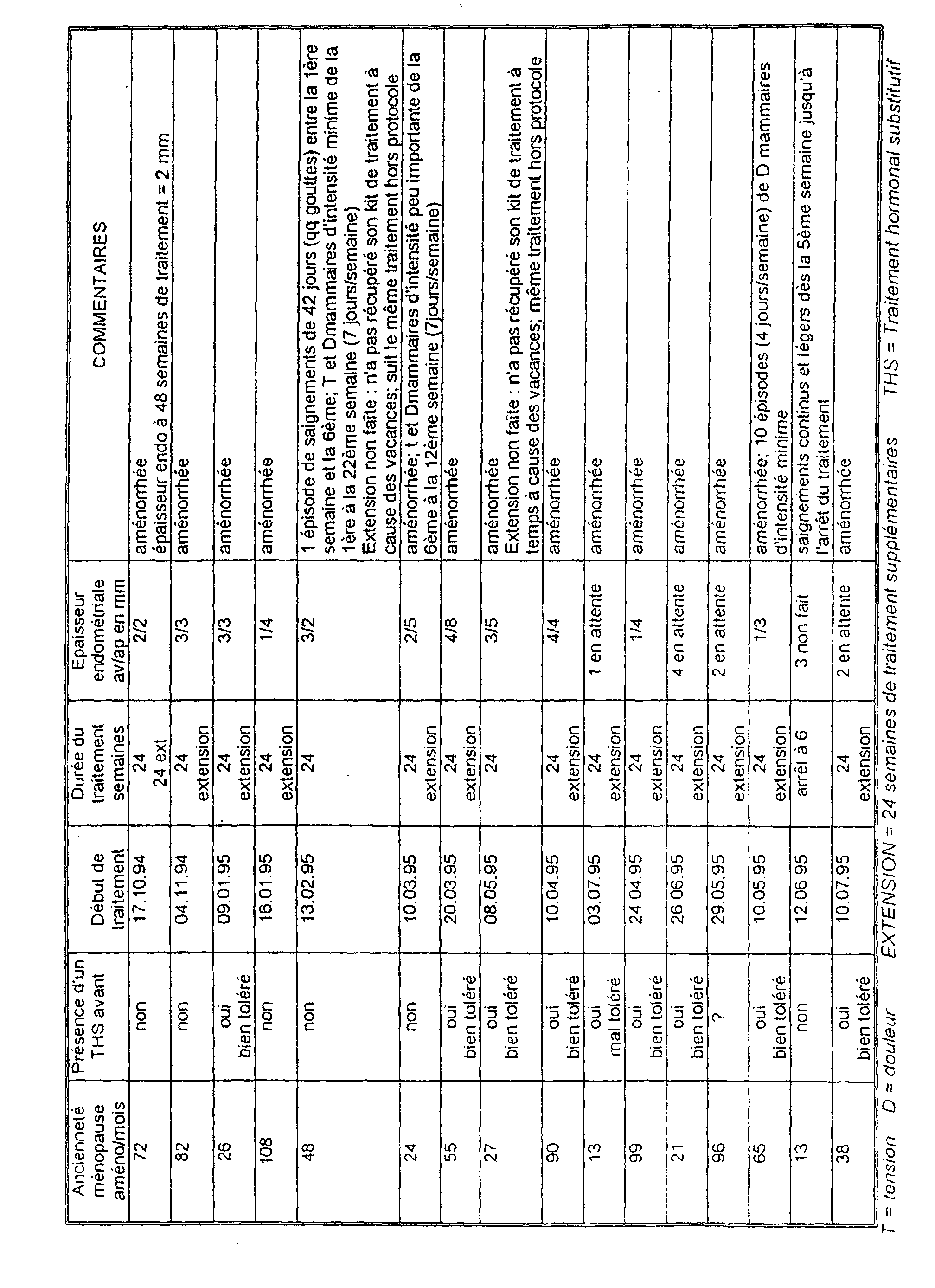

### CONCLUSION

Sur 16 patientes traitées :
- 1 sortie d'essai, soit 6 %
- 15 fins d'essai à 24 semaines, soit 94 %
- 13 extensions de traitement (24 semaines supplémentaires) 81 %
Les deux extensions non faites : dues à des causes indépendantes du traitement, les patientes continuent le même traitement hors protocole.

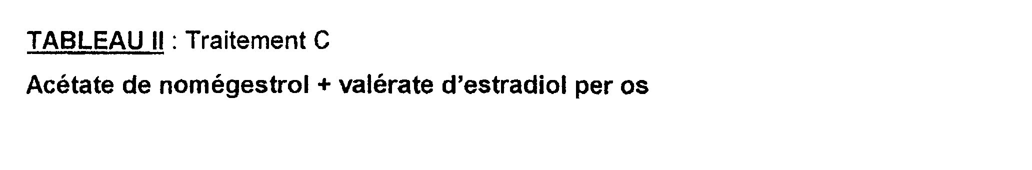

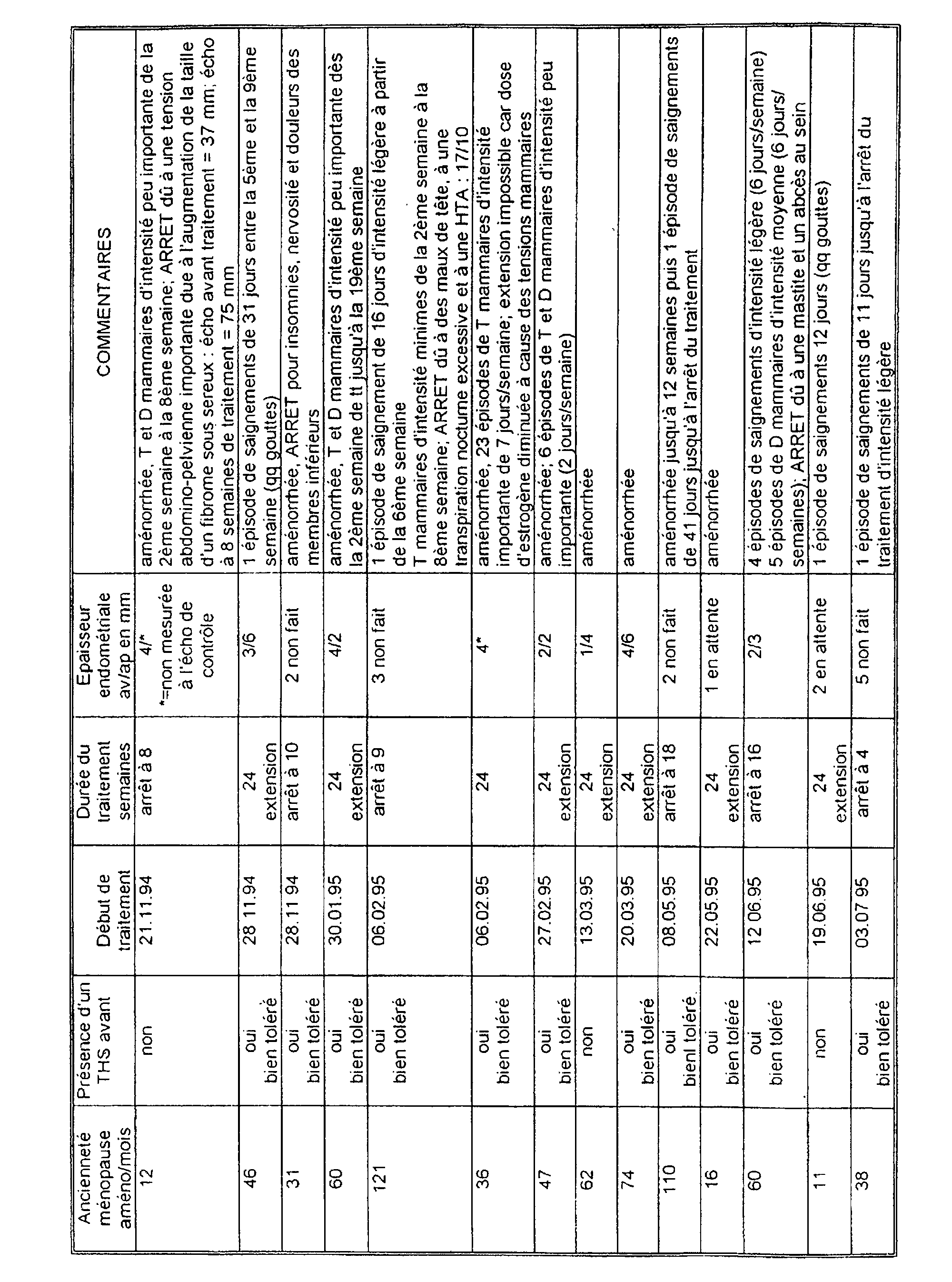

### CONCLUSION

Sur 14 patientes traitées :
- 6 sorties d'essai, soit 43 %
- 8 fin d'essai à 24 semaines, soit 57 %
- 7 extensions de traitement (24 semaines supplémentaires), soit 50 %
**% d'aménorrhée** (soit aucun épisode de saignement pendant 24 semaines) = 43 %

### RESULTATS II

### A - OBSERVANCE

Bien qu'il n'existe pas de différence significative entre les deux groupes A et C de traitement, on observe avec le traitement A, un plus faible nombre de jours d'oubli sur l'ensemble des 24 semaines de l'étude.

### B - TOLERANCE CLINIQUE ENDOMETRIALE

Le pourcentage d'aménorrhée absolue le plus important est retrouvé dans le groupe A, la différence étant significative dans la phase II (13ème à 24ème semaine de traitement). Comme cela est décrit dans la littérature, le pourcentage d'aménorrhée augmente avec le temps; ainsi, pour le groupe C, il est de 35,3 % au cours des 12 premières semaines de traitement, et de 46,1 % au cours des 12 dernières.

Les tableaux III, IV et V ci-joints, illustrent les résultats obtenus.

### AMENORRHEE

### Analyse en intention de traiter

**TABLEAU III:**

| **Phase I / semaines 1 à 12** | | | | | | | |
|---|---|---|---|---|---|---|---|
| | TOTAL | | GROUPE A | | GROUPE C | | P |
| | **N** | % | **N** | % | **N** | % | |
| Aménorrhée | | | | | | | |
| oui | 19 | 37,2 % | 9 | 50 % | 6 | 35,3 % | |
| non | 32 | 62,7 % | 9 | 50 % | 11 | 64,7 % | 0,316 |

| Spottings | | | | | | | |
|---|---|---|---|---|---|---|---|
| oui | 32 | 62,7 % | 9 | 50 % | 11 | 64,7 % | |
| non | 19 | 37,2 % | 9 | 50 % | 6 | 35,3 % | 0,316 |
| *Aucune patiente n'a eu de métrorragies au cours de la phase I* | | | | | | | |

| | TOTAL | | GROUPE A | | GROUPE C | | p |
|---|---|---|---|---|---|---|---|
| | **N** | moy±sem (min:max) | **N** | moy±sem (min:max) | **N** | moy±sem (min:max) | |
| Durée totale de saignements (Jrs) | 51 | 9,1±2,1 0:70 | 18 | 9,1±4,5 0:70 | 17 | 8,9±2,7 0:31 | 0,412 |
| Intensité moyenne | 51 | 0,8±0,1 0:2 | 18 | 0,7±0,2 0:2 | 17 | 0,9±0,20 0:2,5 | 0,446 |
| Nbre de semaines de saignement | 51 | 2,1±0,4 0:10 | 18 | 1,8±0,7 0:10 | 17 | 2,1±0,5 0:7 | 0,552 |
| Nombre total d'épisodes | 51 | 1,2±0,2 0:6 | 18 | 1±0,3 0:4 | 17 | 1,2±0,4 0:6 | 0,434 |

**TABLEAU IV:**

| **Phase II / semaines 13 à 24** | | | | | | | |
|---|---|---|---|---|---|---|---|
| | TOTAL | | GROUPE A | | GROUPE C | | P |
| | **N** | % | **N** | % | **N** | % | |
| Aménorrhée | | | | | | | |
| oui | 20 | 42,5% | 12 | 66,7 % | 6 | 46,1% | |
| non | 27 | 57,4% | 6 | 33,3 % | 7 | 53,8 % | 0,006 |

| Spottings | | | | | | | |
|---|---|---|---|---|---|---|---|
| oui | 27 | 57,4 % | 6 | 33,3 % | 7 | 53,8 % | |
| non | 20 | 42,5 % | 12 | 66,7 % | 6 | 46,1 % | 0,006 |
| *Aucune patiente n'a eu de métrorragies au cours de la phase II* | | | | | | | |

| | TOTAL | | GROUPE A | | GROUPE C | | |
|---|---|---|---|---|---|---|---|
| | **N** | moy±sem (min:max) | **N** | moy±sem (min:max) | **N** | moy±sem (min:max) | P |
| Durée totale de saignements (Jrs) | 47 | 13,9±3,1 0:75 | 18 | 6,2±3,3 0:42 | 13 | 18,5±7,7 0:75 | 0,013 |
| Intensité moyenne | 47 | 0,9±0,1 0:2 | 18 | 0,6±0,2 0:2,33 | 13 | 1,0±0,3 0:2 | 0,055 |
| Nbre de semaines de saignement | 47 | 2,9±0,6 0:12 | 18 | 1,3±0,6 0:9 | 13 | 3,3±1,2 0:11 | 0,007 |
| Nombre total d'épisodes | 47 | 1,3±0,3 0:7 | 18 | 0,6±0,3 0:6 | 13 | 1,1±0,5 0:7 | 0,002 |

**TABLEAU V**

| Δ % entre C1 et C3 | TOTAL | | GROUPE A | | GROUPE C | | P |
|---|---|---|---|---|---|---|---|
| | **N** | moy±sem (min:max) | **N** | moy±sem (min:max) | **N** | moy±sem (min:max) | |
| S.G.P.T (UI/I) | 43 | -23,1%±5,2% | 17 | -19,0%±3,8% | 11 | -31,2%±13,2% | 0,936 |
| | | -88,2%:85,7% | | -50%:7,1% | | -88,2%:29,4% | |
| F.S.H | 45 | -74,1%±4,9% | 18 | -72,2%±5,5% | 12 | -78,2%±9,6% | 0,405 |
| | | -98,4%:69,2% | | -98%:24,8% | | -98,4%:22,8% | |
| Estradiol (pg/ml) | 40 | 432%±68,5% | 15 | 567%±118,7% | 10 | 609%±163,6% | 0,036 |
| | | -54%:1640% | | -16%:1320% | | -54,3%:1640% | |
| *S. G.P.T = Sérum Glutamic Pyruvic Transaminase* | | | | | | | |
| *(ou A.L.A.T. = Alanine Aminotransferase Transaminase)* | | | | | | | |
| *F.S.H = Follicle-Stimulating Hormone* | | | | | | | |

La variation relative du taux d'estradiol est assez importante dans les deux groupes (Δ% = 567% dans le groupe A et 609% dans le groupe C), p = 0,04

Le tableau VI illustre une autre étude effectuée. Dans cette autre étude, il est intéressant de noter qu'avec l'acétate de nomégestrol, le pourcentage de patientes en aménorrhée absolue (toutes formes d'estrogénothérapie confondues) est plus important dès le 3ème mois de traitement : 42,5 % contre 33,3 %. Dans l'étude citée ci-dessus, il faut attendre le 12ème mois de traitement pour obtenir ce pourcentage de 42 % de patientes en aménorrhée obtenu ici dès 3 mois, alors que les populations sont comparables en terme d'âge, de poids et d'ancienneté de la ménopause. De plus, il existe dans l'étude précédente, un effet estrogène que l'on ne retrouve pas dans cette autre étude. En revanche, cette étude a mis en évidence un effet dose du progestatif au cours des 9 derniers mois de traitement (moins la dose de progestatif est importante, meilleur est le contrôle du cycle).

Enfin, il est intéressant de noter qu'il n'existe pas de corrélation entre l'existence d'une aménorrhée et l'épaisseur endométriale à 6 mois mesurée par échographie endovaginale; cette épaisseur variant de +1,6 mm en moyenne au cours des 6 mois dans les 2 groupes de traitement.

**TABLEAU VI**

| **Caractéristiques des patientes** | | | | | | | |
|---|---|---|---|---|---|---|---|
| | TOTAL | | GROUPE A | | GROUPE C | | P |
| | **N** | moy±sem (min:max) | **N** | moy±sem (min:max) | **N** | moy±sem (min:max) | |
| Age | 54 | 54,9±0,6 45:64 | 19 | 53,9±0,8 48:60 | 17 | 54,9±1,1 45:63 | 0,321 |
| Ancienneté de l'aménorrhée(mois) | 54 | 56,1±5,0 7:134 | 19 | 48,5±7,7 12:108 | 17 | 50,7±7,7 11:121 | 0,309 |
| Poids (kg) | 54 | 60±1,1 42:85 | 19 | 61,6±1,2 51:70 | 17 | 60,8±2,2 12:76 | 0,149 |
| Taille (m) | 54 | 1,61±0,01 1,47:1,75 | 19 | 1,62±0,01 1,57:1,75 | 17 | 1,61±0,02 1,47:1,75 | 0,449 |
| Indice de Quetelet (kg/m²) | 54 | 23,1±0,4 17,1:31,2 | 19 | 23,3±0,4 19,7:25,6 | 17 | 23,5±0,7 17,5:28,7 | 0,3182 |
| PAS (mmHg) | 54 | 123,9±1,5 100:140 | 19 | 127,9±2,5 110:140 | 17 | 121,2±2,5 110:140 | 0,136 |
| PAD (mmHg) | 54 | 74,6±1,2 60:90 | 19 | 76,8±2 60:90 | 17 | 73,5±2,3 60:90 | 0,386 |

| T.H.S | TOTAL | | GROUPE A | | GROUPE C | | P |
|---|---|---|---|---|---|---|---|
| | **N** | % | **N** | % | **N** | % | |
| THS antérieurs | | | | | | | |
| oui | 17 | 31,5 % | 9 | 47,4 % | 14 | 82,3 % | |
| non | 37 | 68,5 % | 10 | 52,6 % | 8 | 17,7 % | 0,046 |
| *THS = Traitement Hormonal Substitutif* | | | | | | | |
| *PAS = Pression Artérielle Systolique* | | | | | | | |
| *PAD = Pression Artérielle Diastolique* | | | | | | | |

## Revendications

1. Utilisation d'une composition hormonale estroprogestative, en vue de la réalisation d'un médicament destiné à traiter les carences estrogéniques, tout en évitant la survenue de règles, et à prévenir l'ostéoporose et les maladies cardiovasculaires chez la femme ménopausée, **caractérisée en ce qu'**on administre simultanément d'une manière continue de 21 à 25 jours par mois, une dose allant de 0,5 à 3 mg d'estrogène par prise unitaire et une dose allant de 1,5 à 3,75 mg et d'un dérivé de la 19-norprogestérone par prise unitaire, en association ou en mélange avec un ou plusieurs excipients non toxiques, inertes, pharmaceutiquement acceptables.

2. Utilisation d'une composition hormonale estroprogestative, selon la revendication 1, **caractérisée en ce qu'**elle est administrable par voie orale.

3. Utilisation d'une composition hormonale estroprogestative selon la revendication 1, dans laquelle l'estrogène est l'estradiol libre ou estérifié, ou des estrogènes conjugués équins.

4. Utilisation d'une composition hormonale estroprogestative selon la revendication 1 ou la revendication 3, dans lesquelles l'estrogène est un ester d'estradiol.

5. Utilisation d'une composition hormonale estroprogestative selon la revendication 1 ou la revendication 3, dans lesquelles l'estradiol libre est présent de préférence à une dose de 1,5 mg par prise unitaire.

6. Utilisation d'une composition hormonale estroprogestative selon la revendication 1 ou la revendication 3, dans laquelle l'ester d'estradiol est présent de préférence à une dose de 2 mg par prise unitaire.

7. Utilisation d'une composition hormonale estroprogestative selon la revendication 1 ou la revendication 3, dans laquelle l'estrogène conjugué équin est présent de préférence à une dose de 0,625 mg par prise unitaire.

8. Utilisation d'une composition hormonale estroprogestative selon la revendication 1, dans laquelle le dérivé de la 19-norprogestérone est l'acétate de nomégestrol.

9. Utilisation d'une composition hormonale estroprogestative selon la revendication 1 ou la revendication 8, dans laquelle l'acétate de nomegestrol est présent à une dose allant de 1,5 à 3,75 mg par prise unitaire.

10. Utilisation d'une composition hormonale estroprogestative selon la revendication 1 ou la revendication 8, dans lesquelles l'acétate de nomegestrol est présent à une dose de 2,5 mg par prise unitaire.

## Patentansprüche

1. Anwendung eines hormonalen oestroprogestativen Präparats zur Darstellung eines Medikaments, welches für die Behandlung östrogener Mangelzustände unter Vermeidung des unverhofften Eintretens der Menstruation und zur Vorbeugung vor Osteoporose und kardiovaskulärer Erkrankungen bei der Frau im Klimakterium bestimmt ist, **dadurch gekennzeichnet, dass** man gleichzeitig auf kontinuierliche Weise über 21 bis 25 Tage pro Monat eine Dosis Östrogen, die von 0,5 bis 3 mg pro Dosiereinheit reicht, und eine Dosis eines Derivats von 19-Norprogesteron, die von 1,5 bis 3,75 mg pro Dosiereinheit reicht, in Verbindung oder im Gemisch mit einer oder mehreren nichttoxischen, inerten pharmazeutisch-verträglichen Träger verabreicht.

2. Anwendung eines hormonalen oestroprogestativen Präparats gemäß Anspruch 1, **dadurch gekennzeichnet, dass** dieses oral verabreichbar ist.

3. Anwendung eines hormonellen oestroprogestativen Präparats gemäß Anspruch 1, bei welchem das Östrogen freies oder verestertes Östradiol ist oder eine Pferde konjugierte Östrogen sind.

4. Anwendung eines hormonellen oestroprogestativen Präparats gemäß Anspruch 1 oder Anspruch 3, bei welchem das Östrogen ein Östradiolester ist.

5. Anwendung eines hormonellen oestroprogestativen Präparats gemäß Anspruch 1 oder Anspruch 3, bei welchem das freie Östradiol vorzugsweise in einer Dosis von 1,5 mg pro Dosiereinheit vorliegt.

6. Anwendung eines hormonellen oestroprogestativen Präparats gemäß Anspruch 1 oder Anspruch 3, bei welchem der Östradiolester vorzugsweise in einer Dosis von 2 mg pro Dosiereinheit vorliegt.

7. Anwendung eines hormonalen oestroprogestativen Präparats gemäß Anspruch 1 oder Anspruch 3, bei welchem das Pferde konjugierte Östrogen vorzugsweise in einer Dosis von 0,625 mg pro Dosiereinheit vorliegt.

8. Anwendung eines hormonalen oestroprogestativen Präparats gemäß Anspruch 1, bei welchem das Derivat des 19-Norprogesterons Nomegestrolacetat ist.

9. Anwendung eines hormonalen oestroprogestativen Präparats gemäß Anspruch 1 oder Anspruch 8, bei welchem das Nomegestrolacetat in einer Dosis vorliegt, die von 1,5 bis 3,75 mg pro Dosiereinheit reicht.

10. Anwendung eines hormonalen oestroprogestativen Präparats gemäß Anspruch 1 oder Anspruch 8, bei welchem das Nomegestrolacetat in einer Dosis von 2,5 mg pro Dosiereinheit vorliegt.

## Claims

1. Use of an estroprogestative hormonal composition intended for the achievement of a drug for the treatment of estrogenic deficiencies while avoiding the occurrence of mendes, and for prevent ring osteoporosis and cardiovascular disease in the menopaused woman, wherein they are given simultaneously and continuously during 21 to 25 days a months a dose ranging from 0.5 to 3 mg estrogen per mint dosage and a dosis ranging from 1.5 to 375 mg of a derivative of 19-norprogesterone per unit dosage in combination or admixture with one or several non toxic, inert, pharmaceutically-acceptable carriers.

2. Use of an estroprogestative hormonal composition according to claim 1, wherein the composition may be given through oral way.

3. Use of an estroprogestative hormonal composition according to claim 1, wherein the estrogen is free or esterified estradiol or equine conjugated estrogens.

4. Use of an estroprogestative hormonal composition according to claim 1 or claim 3, wherein the estrogen is an ester of estradriol.

5. Use of an composition according to claim 1 or to claim 3, where the free estradiol is preferably present at a dosing of 1,5 mg per unit dosage.

6. Use of an estroprogestative hormonal composition according to claim 1 or claim 3, wherein the estradiol ester is preferably present at a dosis of 2 mg per unit dosage.

7. Use of an estroprogestative hormonal composition according to claim 1 or claim 3, wherein the equine conjugated estrogen is preferably present at a dosis of 0,625 mg per unit dosage.

8. Use of an estroprogestative hormonal composition according to claim 1 wherein the derivative of 19-norprogesterone is nomegestrol acetate.

9. Use of an estroprogestative hormonal composition according to claim 1 or claim 8, wherein nomegestrol acetate is present in an amount ranging from 1,5 to 3.75 mg per unit dosage.

10. Use of an estroprogestative hormonal composition according to claim 1 or claim 3 wherein nomegestrol acetate is present at an amount of 2.5 mg per unit dosage.
